(19) Europäisches Patentamt
European Patent Office
Office européen des brevets

(11) EP 3 708 082 A1

(12) EUROPEAN PATENT APPLICATION
published in accordance with Art. 153(4) EPC

(43) Date of publication:
16.09.2020 Bulletin 2020/38

(51) Int Cl.:
A61B 6/00 (2006.01)     A61B 6/03 (2006.01)

(21) Application number: 18875286.9

(22) Date of filing: 12.09.2018

(86) International application number:
PCT/JP2018/033731

(87) International publication number:
WO 2019/092981 (16.05.2019 Gazette 2019/20)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 08.11.2017  JP 2017215898

(71) Applicant: Canon Kabushiki Kaisha
Tokyo 146-8501 (JP)

(72) Inventor: MACHIDA, Yoshihito
Tokyo 146-8501 (JP)

(74) Representative: TBK
Bavariaring 4-6
80336 München (DE)

(54) IMAGE PROCESSING DEVICE, IMAGE PROCESSING METHOD, RADIATION IMAGING DEVICE, AND METHOD AND PROGRAM FOR CONTROLLING RADIATION IMAGING DEVICE

(57) An image processing apparatus acquires a plurality of radiation images by radiation imaging with a plurality of energies, and detects, based on pixel values of the plurality of radiation images, whether there is any abnormal pixel in at least one of the plurality of radiation images.

FIG. 1

EP 3 708 082 A1

**Description**

TECHNICAL FIELD

The present invention relates to a radiation imaging technique and, more particularly, to the detection and correction of abnormal pixels upon acquisition of a plurality of images with different energies.

BACKGROUND ART

Currently, a radiation imaging apparatus using an FPD (Flat Panel Detector) formed from a semiconductor material has been popularized as an imaging apparatus used for medical image diagnosis or non-destructive inspection using X-rays. Such a radiation imaging apparatus is used as a digital imaging apparatus for still-image imaging like general imaging or moving-image imaging like fluoroscopic imaging in, for example, medical image diagnosis. In addition, this apparatus is used for dual energy imaging and the like that acquire radiations having two different energies.

An indirect FPD that converts and detects a radiation quantum into visible light with a phosphor has its own problem. The phosphor does not convert all radiation into visible light, and a photoelectric conversion unit probabilistically absorbs radiation transmitted through the phosphor. This phenomenon generates a large amount of electric charges several ten to several hundred times (depending on the arrangement of the sensor) that generated when radiation is converted into visible light. Accordingly, it is known that outputs from pixels having experienced this phenomenon become higher than normal and generate high-luminance spot noise to cause a deterioration in image quality.

PTL 1 discloses a technique of extracting such noise (abnormal pixels). More specifically, PTL 1 discloses a method of determining noise by dividing an image (first image) obtained by a first readout operation within the irradiation time of radiation by an image (second image) obtained by a second readout operation after the first readout operation and determining whether the obtained value is a predetermined value.

CITATION LIST

PATENT LITERATURE

PTL 1: Japanese Patent Laid-Open No. 2014-183475

SUMMARY OF INVENTION

TECHNICAL PROBLEM

When an FPD is used in a dual energy system as well, the above noise is generated. In this case, for example, according to the method disclosed in PTL 1, because the rate is not constant even without noise, it is difficult to extract noise.

The present invention has been made in consideration of the above problem, and provides a technique for efficiently detecting whether there is any abnormal pixel in a plurality of images obtained by radiation imaging with a plurality of energies.

SOLUTION TO PROBLEM

As a means for achieving the above object, the image processing apparatus according to the present invention has the following arrangement. That is, the apparatus includes image acquisition means for acquiring a plurality of radiation images by radiation imaging with a plurality of energies and detection means for detecting, based on the pixel values of the plurality of radiation images, whether there is any abnormal pixel in at least one of the plurality of radiation images.

ADVANTAGEOUS EFFECTS OF INVENTION

It is possible to efficiency detect whether there is any abnormal pixel in a plurality of images obtained by radiation imaging with a plurality of energies.

Other features and advantages of the present invention will be apparent from the following description made with reference to the accompanying drawings. Note that the same reference numerals denote the same or similar components throughout the accompanying drawings.

BRIEF DESCRIPTION OF DRAWINGS

The accompanying drawings, which are incorporated in and constitute a part of the specification, illustrate presently preferred embodiments of the invention, and together with the general description given above and the detailed description of the preferred embodiments given below, serve to explain the principles of the invention.

Fig. 1 is a block diagram showing an example of the arrangement of a radiation imaging system 10 according to the first embodiment;

Fig. 2 is a block diagram showing an example of the hardware arrangement of an image processing apparatus 100;

Fig. 3 is a block diagram showing an example of the arrangement of a radiation detection apparatus 102;

Fig. 4 is a timing chart showing the processing timing of the radiation detection apparatus 102;

Fig. 5 is a block diagram showing an example of the functional arrangement of a substance information calculation unit 103;

Fig. 6 is a view showing an example of a conversion table according to the first embodiment;

Fig. 7 is a view showing an example of an image after pixel adjustment by a pixel adjustment unit 506;

Fig. 8A is a view showing a pattern of pixels around an abnormal pixel;

Fig. 8B is a view showing a pattern of pixels around an abnormal pixel;

Fig. 9A is a view showing another pattern of pixels around an abnormal pixel;

Fig. 9B is a view showing another pattern of pixels around an abnormal pixel;

Fig. 9C is a view showing another pattern of pixels around an abnormal pixel;

Fig. 10 is a view showing an example of a conversion table according to modification 1-3;

Fig. 11 is a view showing an example of a conversion table according to modification 1-4;

Fig. 12 is a block diagram showing an example of the arrangement of a radiation imaging system 1200 according to the second embodiment;

Fig. 13 is a block diagram showing the functional arrangement of an abnormal pixel detection unit 1201;

Fig. 14 is a view showing an example of an abnormal pixel determination result;

Fig. 15 is a view showing an example of a conversion table according to modification 1-1; and

Fig. 16 is a block diagram showing the functional arrangement of an abnormal pixel correction unit 1202.

DESCRIPTION OF EMBODIMENTS

The present invention will be described in detail below based on each embodiment as an example with reference to the accompanying drawings. Note that the arrangement of each embodiment described below is merely an example, and the present invention is not limited to any arrangement shown in the accompanying drawings.

[First Embodiment]

(Arrangement of Radiation Imaging System 10)

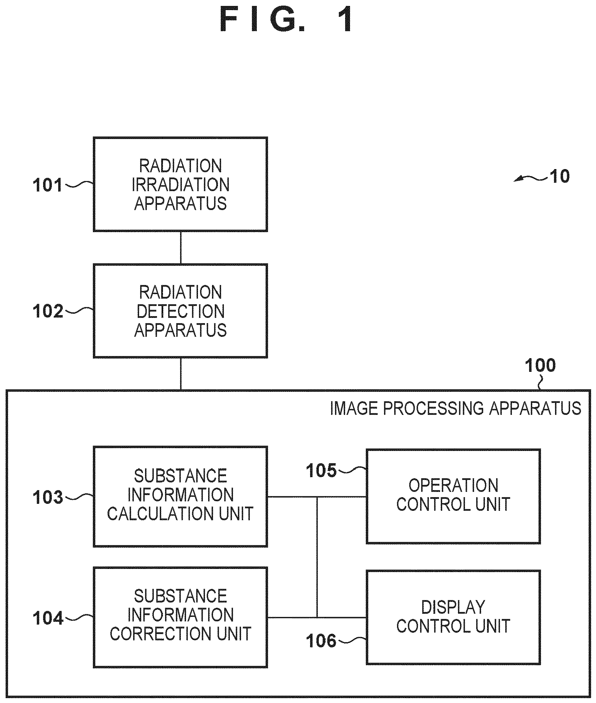

Fig. 1 shows an example of the arrangement of a radiation imaging system 10 according to the first embodiment. The radiation imaging system 10 is constituted by a radiation irradiation apparatus 101, a radiation detection apparatus 102, and an image processing apparatus 100. The radiation irradiation apparatus 101 irradiates an object (not shown) with radiation. The radiation detection apparatus 102 acquires images obtained when applied radiations having two different types of energies are transmitted through an object. Note that a method of acquiring images by using radiations having a plurality of different energies according to the first embodiment will be described later. The radiation detection apparatus 102 and the image processing apparatus 100 can also constitute a radiation imaging apparatus as one apparatus.

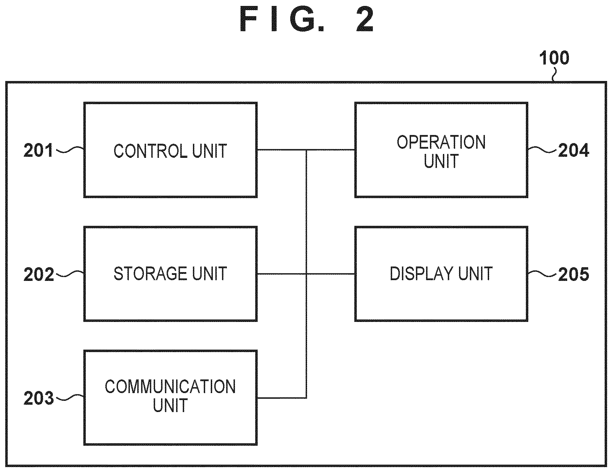

The functional arrangement of the image processing apparatus 100 is constituted by a substance information calculation unit 103, a substance information correction unit 104, an operation control unit 105, and a display control unit 106. The substance information calculation unit 103 calculates substance information based on the images obtained by two different types of energies, and detects whether there is any abnormal pixel as noise. Note that substance information according to this embodiment is an effective atomic number corresponding to a substance. The substance information correction unit 104 corrects the substance information calculated by the substance information calculation unit 103 based on an abnormal pixel detection result. The operations of the substance information calculation unit 103 and the substance information correction unit 104 will be described later. The operation control unit 105 converts the operation of the operator which is accepted by an operation unit 204 (Fig. 2) into a control signal, and transfers the signal to the substance information calculation unit 103, the substance information correction unit 104, and the display control unit 106. The display control unit 106 performs control to cause a display unit 205 (Fig. 2) to display the information obtained by the substance information correction unit 104 under the control of the operation control unit 105.

Fig. 2 shows an example of the hardware arrangement of the image processing apparatus 100. The hardware arrangement of the image processing apparatus 100 is constituted by a control unit 201, a storage unit 202, a communication unit 203, the operation unit 204, and the display unit 205. The control unit 201 is, for example, a CPU (Central Processing Unit), and controls the operation each constituent element. The storage unit 202 is constituted by a ROM (Read Only Memory) and a RAM (Random Access Memory). The storage unit 202 can store control instructions, that is, programs. For example, the storage unit 112 can also be used as a work memory at the time of execution of a program and used to temporarily save data. The communication unit 203 performs control to communicate with an external apparatus. The operation unit 204 accepts the operation of the operator. The display unit 205 performs various kinds of display. The display unit 205 is formed from, for example, a display panel that displays image data. The display panel may be formed from a display system such as an LCD (Liquid Crystal Display) system, plasma system, or organic EL system or may be a projection type display device that projects on a wall surface.

(Method of Acquiring Images Using Radiations Having Two Different Types of Energies)

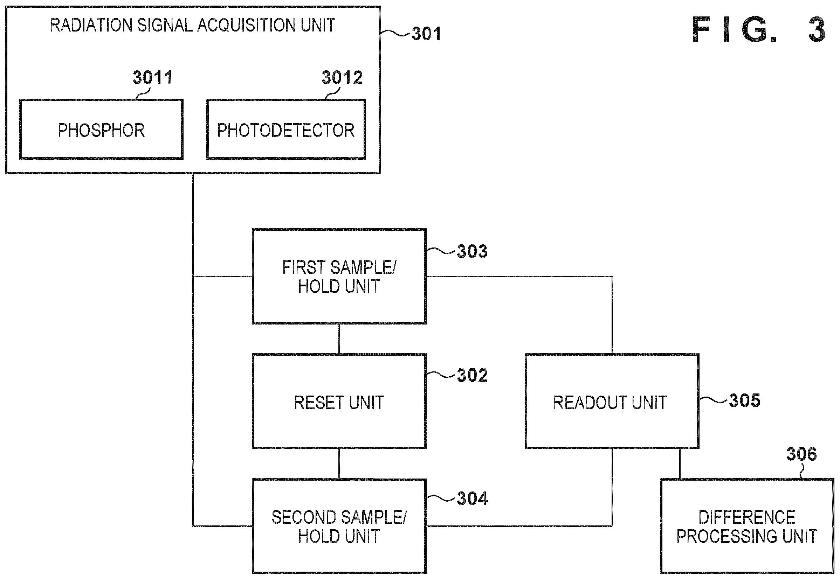

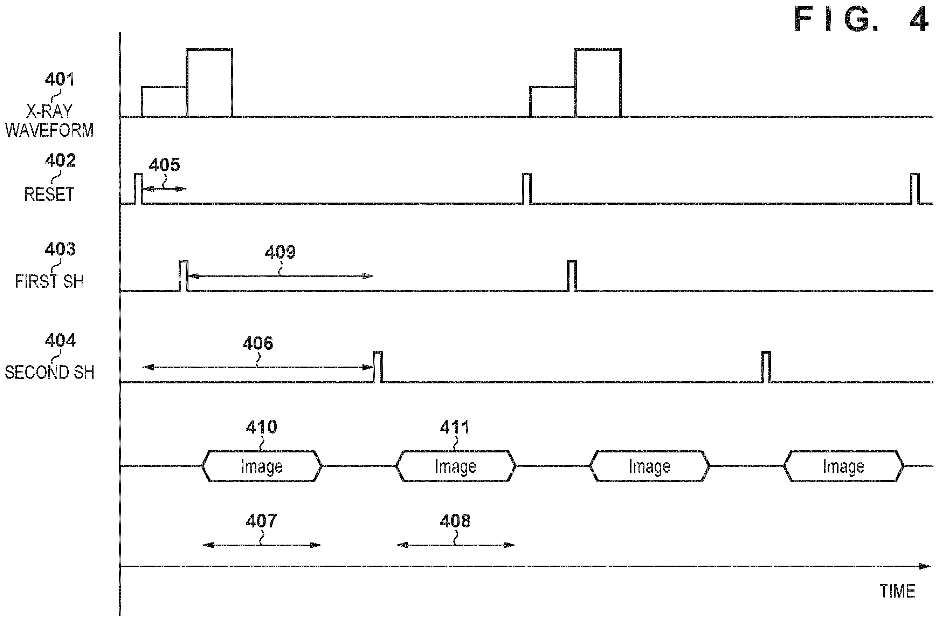

A method of acquiring images by using two different types of energy radiations in the radiation detection apparatus 102 will be described in detail with reference to Figs. 3 and 4. This embodiment will exemplify a case in which a plurality of images are acquired by using the image acquisition function disclosed in PTL 2. Although the embodiment exemplifies a method of acquiring two types of radiation images by using two types of radiations using a sample/hold operation at high speed, the embodiment can also be applied to a method of acquiring images by performing two exposures with different tube voltages without performing any sample/hold operation.

Fig. 3 shows an example of the arrangement of the radiation detection apparatus 102. The radiation detection apparatus 102 is constituted by a radiation signal acquisition unit 301, a reset unit 302, a first sample/hold unit 303, a second sample/hold unit 304, a readout unit 305, and a difference processing unit 306. The radiation signal acquisition unit 301 is constituted by a phosphor 3011 and a photodetector 3012. The radiation signal acquisition unit 301 converts radiation into electric charges in an amount proportional to the radiation and accumulates the electric charges. More specifically, the phosphor 3011 converts radiation into light. The photodetector 3012 detects the light converted by the phosphor 3011, generates electric charges corresponding to the light (converts light into electric charges), and accumulates the electric charges. The reset unit 302 resets the electric charges accumulated in the radiation signal acquisition unit 301. The first sample/hold unit 303 and the second sample/hold unit 304 accumulate the electric charges acquired by the radiation signal acquisition unit 301. The readout unit 305 reads out the electric charges accumulated by the first sample/hold unit 303 and the second sample/hold unit 304, and acquires electric charges as pixel values. The difference processing unit 306 reads out pixel values by using the readout unit 305, and performs difference processing of the pixel values.

Fig. 4 is a timing chart showing the timings of irradiation with radiation by the radiation irradiation apparatus 101 and the detection operation of the radiation detection apparatus 102. Fig. 4 shows the tube voltage waveform (X-ray waveform 401) of radiation applied by the radiation irradiation apparatus 101, the operation timing (reset 402) of the reset unit 302, the acquisition timing (first SH 403) of the first sample/hold unit 303, and the acquisition timing (second SH 404) of the second sample/hold unit 304. A period 405 is the period from a reset operation to the acquisition timing of the first sample/hold operation. A period 406 is the period from a reset operation to the acquisition timing of the second sample/hold operation. A period 407 indicates an image readout timing after the first sample/hold operation. A period 408 indicates an image readout timing after the second sample/hold operation.

A procedure for the method of acquiring radiation images by this imaging apparatus using two types of energy radiations will be described with reference to the timing chart of Fig. 4. First of all, the reset unit 302 resets the electric charges accumulated by the radiation signal acquisition unit 301. The radiation irradiation apparatus 101 then applies radiation. The radiation signal acquisition unit 301 converts the radiation into electric charges and accumulates the electric charges. The first sample/hold unit 303 reads the electric charges accumulated by the radiation signal acquisition unit 301. The readout unit 305 then reads the image accumulated in the first sample/hold unit 303. In this case, the readout unit 305 reads a pixel value 410 proportional to the dose of radiation applied in the period 405.

Subsequently, the second sample/hold unit 304 reads the amount of electric charges accumulated by the radiation signal acquisition unit 301. In this case, the second sample/hold unit 304 reads a pixel value 411 proportional to the dose of radiation applied in the period 406. Subtracting the pixel value 410 from the pixel value 411 by using the difference processing unit 306 can obtain a pixel value in a period 409. The period 405 is a period in which the tube voltage is low. The period 409 is a period in which the tube voltage is high or no radiation is applied. Accordingly, the image in the period 405 differs in energy from the image in the period 409. Properly setting sample/hold timings in this manner can quickly acquire images using radiations having a plurality of different energies, and can reduce the influence of movement on a moving image such as a fluoroscopic image.

(Methods of Detecting Abnormal Pixel and Calculating Substance Information)

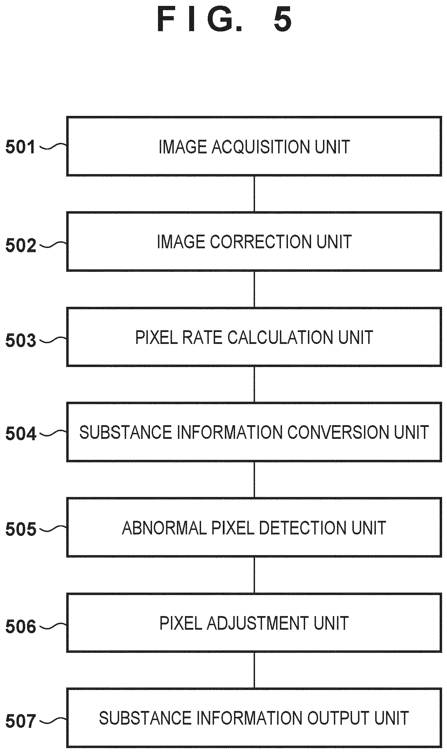

Methods of detecting an abnormal pixel and calculating substance information in the substance information calculation unit 103 will be described in detail next. An abnormal pixel is a pixel generated when radiation that is not converted into light by the phosphor 3011 enters the photodetector 3012. Fig. 5 shows an example of the functional arrangement of the substance information calculation unit 103. The substance information calculation unit 103 is constituted by an image acquisition unit 501, an image correction unit 502, a pixel rate calculation unit 503, a substance information conversion unit 504, an abnormal pixel detection unit 505, a pixel adjustment unit 506, and a substance information output unit 507.

The image acquisition unit 501 acquires a plurality of radiation images by radiation imaging with a plurality of energies from the radiation detection apparatus 102. In this embodiment, the image acquisition unit 501 acquires radiation images obtained by radiation imaging with two types of energies, that is, a radiation image (high-energy radiation image) obtained by radiation imaging with higher energy and a radiation image (low-energy radiation image) obtained by radiation imaging with lower energy. The image correction unit 502 performs correction for two types of radiation images acquired by the image acquisition unit 501. This correction can be performed based on equation (1) given below.

$$I_{correct} = \frac{I_{input} - I_{dark}}{I_{air} - I_{dark}} \qquad ...(1)$$

where $I_{correct}$ is a corrected image, $I_{input}$ is an image acquired by irradiating an object in a given state with radiation, lair is an image separately acquired under the same condition as that under which the image $I_{input}$ is acquired without any object, and Idark is an image separately acquired without irradiation with radiation (the unit is a pixel value for each image). In this embodiment, the image correction unit 502 performs this correction for each of two types of radiation images. Performing such correction makes it possible to visualize the transmittance of radiation irradiating an object.

The pixel rate calculation unit 503 then calculates pixel values of the high-energy radiation image and the low-energy radiation image for each pixel. More specifically, the pixel rate calculation unit 503 calculates the logarithmic rate between the two types of images after correction based on equation (2) given below.

$$I_{rate} = \frac{\log I_h}{\log I_l} \qquad ...(2)$$

where $I_{rate}$ is the logarithmic rate between the two types of corrected images, $I_h$ is an image, of the two types of images, which is acquired with higher energy and corrected according to equation (1), and $I_1$ is an image, of the two types of images, which is acquired with lower energy and corrected according to equation (1).

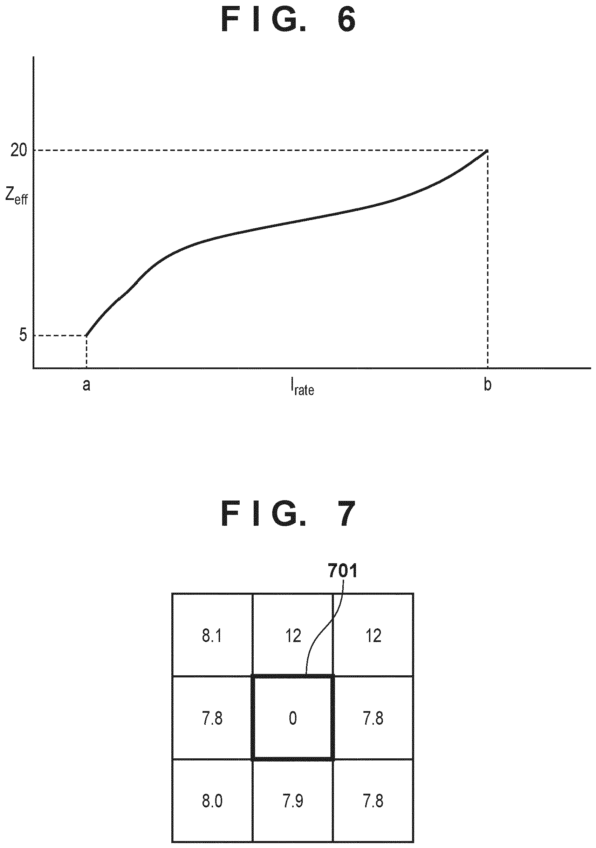

The substance information conversion unit 504 then converts the pixel rate obtained by equation (2) into substance information specifying a substance that can be contained in the object at the time of imaging. As described above, this embodiment uses an effective atomic number corresponding to a substance as substance information. In addition, the embodiment uses a conversion table like that shown in Fig. 6 for conversion into substance information. Fig. 6 shows an example of a conversion table for converting a pixel rate into an effective atomic number in the embodiment. Referring to Fig. 6, the ordinate represents effective atomic number, and the ordinate represents pixel rate. By using this conversion table, the substance information conversion unit 504 calculates (decides) substance information by converting a pixel rate into an effective atomic number. That is, the substance information conversion unit 504 calculates substance information by converting a pixel rate into an effective atomic number. Note that this conversion table can be prepared in advance by performing calculation based on equation (3) given below.

$$I_{rate}(Z_{eff}, E_h, E_l) = \frac{\mu(E_l, Z_{eff})}{\mu(E_h, Z_{eff})} \qquad ...(3)$$

where $Z_{eff}$ is an effective atomic number, $E_h$ is the higher energy (high energy) of two types of energies, $E_1$ is the lower energy (low energy) of the two types of energies, and $I_{rate}(Z_{eff}, E_h, E_l)$ is the logarithmic rate of the two types of images with the effective atomic number $Z_{eff}$, the high energy $E_h$, and the low energy $E_l$. In addition, $\mu(E_h, Z_{eff})$ is a linear attenuation coefficient set when a substance corresponding to $Z_{eff}$ is irradiated with radiation having the high energy $E_h$, $\mu(E_h, Z_{eff})$ is a linear attenuation coefficient set when the substance corresponding to $Z_{eff}$ is irradiated with radiation having the low energy $E_l$. Equations (3) and (2) indicate that the logarithmic rate between the two types of images corresponds to the linear attenuation coefficient rate between the two types of images. The conversion table shown in Fig. 6 can be prepared in advance by calculating equation (3) with respect to effective atomic numbers corresponding to various types of objects as targets. Referring to Fig. 6, effective atomic numbers 5 to 20 are used as effective atomic numbers corresponding to various types of substances as targets. This range corresponds to effective atomic numbers corresponding to substances that can be objects subjected to actual radiation imaging. The operator can separately set this range (values) via the operation unit 204 or automatically depending on the intended use.

The number of combinations of high and low energies for the calculation of equation (3) may be determined in advance in accordance with imaging conditions, setting values such as tube voltages, and the like. In practice, two types of images obtained with high and low energies often have spectrum distributions, but equation (3) is calculated upon approximation of high and low energies to single energy. Accordingly, calibration may be performed such that transmittances are calculated by imaging substances (for example, water and aluminum) having known effective atomic numbers with radiation on the high energy side and radiation on the low energy side, and single energy nearest to the calculated transmittances is set.

Subsequently, the abnormal pixel detection unit 505 detects, based on the pixel values of the plurality of radiation images acquired by the image acquisition unit 501, whether radiation that is not converted into light by the phosphor 3011 has entered the photodetector 3012. That is, the abnormal pixel detection unit 505 determines, based on the pixel values of the plurality of radiation images acquired by the image acquisition unit 501, whether there is any abnormal pixel in at least one of the plurality of radiation images. In this embodiment, the abnormal pixel detection unit 505 detects, based on the pixel value of a high-energy radiation image, the pixel value of a low-energy radiation image, and information concerning a predetermined substance that can be contained in an object at the time of radiation imaging, whether there is any abnormal pixel in any one of the high-energy radiation image and the low-energy radiation image. In this embodiment, if the pixel rate calculated by the pixel rate calculation unit 503 falls outside the rate range between a linear attenuation coefficient set when the substance that can be contained in an object is irradiated with radiation having higher energy and a linear attenuation coefficient set when the substance is irradiated with radiation having lower energy, the abnormal pixel detection unit 505 detects that there is an abnormal pixel.

More specifically, the abnormal pixel detection unit 505 determines whether the pixel rate (logarithmic rate) obtained by equation (2) falls within the range of the conversion table shown in Fig. 6, and detects, based on the determination result, whether there is any abnormal pixel. That is, the abnormal pixel detection unit 505 determines whether the pixel rate falls within the range [a, b] on the abscissa (pixel rate) in Fig. 6. The range [a, b] of pixel rates corresponding to effective atomic numbers 5 to 20 is the range of pixel rates that can be generated at the time of actual imaging. Accordingly, if a pixel rate falls outside the range [a, b], the abnormal pixel detection unit 505 detects that there is an abnormal pixel.

That is, the linear attenuation coefficient excessively decreases or increases due to the influence of an abnormal pixel in one of the high-energy radiation image and the low-energy radiation image, and the abnormal pixel detection unit 505 determines that there is an abnormal pixel. In addition, the magnitude of this linear attenuation coefficient is decided in accordance with the energies of radiations, and hence it is possible to detect whether there is any abnormal pixel, even with different energies between sample/hold operations, by referring to the range of a table corresponding to the combination of energies.

Subsequently, the pixel adjustment unit 506 adjusts pixels with respect to an abnormal pixel, and sets a value that enables to identify the abnormal pixel. Fig. 7 shows an example of an image for which the pixel adjustment unit 506 sets 0 as a value that enables to identify an abnormal pixel. Before adjustment by the pixel adjustment unit 506, the effective atomic numbers converted by using the conversion table in Fig. 6 are assigned to the respective pixels. The pixel adjustment unit 506 performs adjustment to set the pixel value of an abnormal pixel 701 to 0, as shown in Fig. 7. This makes the abnormal pixel 701 have a value different from the pixel values of the normal neighboring pixels, thereby enabling to identify the abnormal pixel. Inputting a pixel value (numerical value) outside the range of effective atomic numbers (that is, 5 to 20) in the conversion table to an abnormal pixel in this manner makes it possible to discriminate whether there is any abnormal pixel among pixels. The substance information output unit 507 outputs the substance information obtained by adjusting the pixels using the pixel adjustment unit 506.

(Method of Correcting Abnormal Pixel)

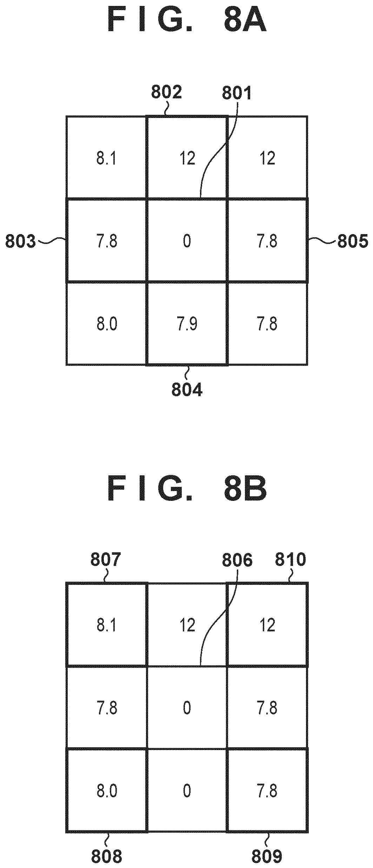

A method of correcting substance information in the substance information correction unit 104 will be described in detail next. In this embodiment, the substance information correction unit 104 corrects substance information of an abnormal pixel detected by the abnormal pixel detection unit 505 by using the neighboring pixels around the pixel. Figs. 8A and 8B show patterns having different neighboring pixels around abnormal pixels when the substance information correction unit 104 corrects substance information of an abnormal pixel by using substance information of pixels adjacent to the abnormal pixel in spatial directions.

Fig. 8A shows a pattern having four normal pixels around an abnormal pixel. In the case shown in Fig. 8A, the substance information correction unit 104 corrects an abnormal pixel 801 by using four neighboring pixels 802 to 803.

For example, the substance information correction unit 104 corrects the substance information of the abnormal pixel 801 by interpolating the pixel value of the abnormal pixel 801 with the average value of the pixel values of the pixels 802 to 805.

Fig. 8B shows a pattern in which all four neighboring pixels around an abnormal pixel are abnormal pixels. In the case shown in Fig. 8B, the substance information correction unit 104 corrects an abnormal pixel 806 by using four neighboring pixels 807 to 810. For example, the substance information correction unit 104 corrects the substance information of the abnormal pixel 806 by interpolating the pixel value of the abnormal pixel 806 with the average value of the pixel values of the pixels 807 to 810.

Although the cases shown in Figs. 8A and 8B use the method of changing pixels to be used for correcting an abnormal pixel based on the state of the abnormal pixel and its neighboring pixels, the manner of using specific pixels for correction is not limited to such a method. For example, referring to Fig. 8A, if the pixel 805 is an abnormal pixel, the pixel 801 may be corrected by using only the pixels 802 to 804 without using the pixel 805. This system can also be configured to allow the operator to separately set pixels to be used for correction via the operation unit 204 or to automatically set them.

As described above, the radiation imaging system 10 according to this embodiment can obtain substance information with reduced noise by efficiently detecting the presence/absence of an abnormal pixel from two image data based on different energies and then performing correction.

[Modification 1-1]

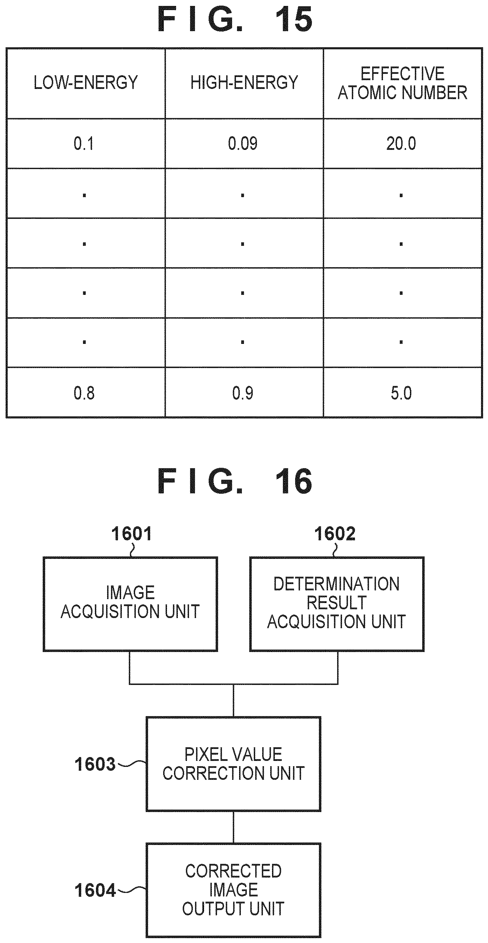

According to the first embodiment, the substance information calculation unit 103 uses a conversion table like that shown in Fig. 6. However, the substance information calculation unit 103 can use a conversion table like that shown in Fig. 15 instead of this table. Fig. 15 shows an example of a conversion table according this modification. Referring to Fig. 15, the first and second columns of the conversion table respectively describe the pixel values of low-energy radiation images and high-energy radiation images, and the third column describes effective atomic numbers. Values described in the conversion table can be calculated in advance based on equation (4) given below.

$$I_{correct} = \int E \, e^{-\mu(E, Z_{eff})t} dE \qquad \qquad ...(4)$$

where $I_{correct}$ is an image corresponding to an image after correction based on equation (1), E is the energy of radiation, $\mu(E, Z_{eff})$ is a linear attenuation coefficient set when a substance corresponding to the effective atomic number $Z_{eff}$ is irradiated with radiation having the energy E, and t is the thickness of the substance.

In this modification, the abnormal pixel detection unit 505 detects whether there is any abnormal pixel, based on whether the combination of the pixel values of two types of radiation images after correction by the image correction unit 502 falls within the range of the conversion table shown in Fig. 15. That is, the abnormal pixel detection unit 505 detects that there is an abnormal pixel when the pixel values of a high-energy radiation image and a low-energy radiation image each do not fall within the range of the pixel values obtained from effective atomic numbers of substances that can be contained in an object at the time of imaging and the two types of energies. In this modification, using a conversion table like that shown in Fig. 15 can perform accurate calculation with respect to radiation having a spectrum distribution.

[Modification 1-2]

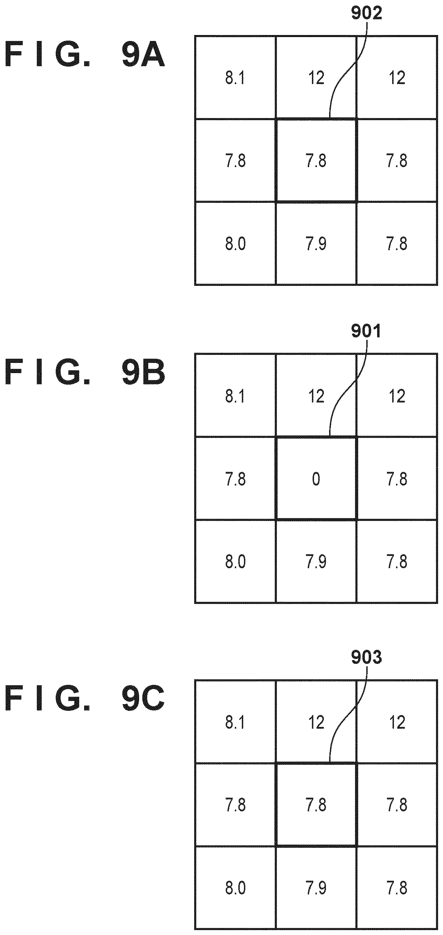

According to the first embodiment, the substance information correction unit 104 uses neighboring pixels in space for the correction of substance information. However, the substance information correction unit 104 may use preceding and succeeding frames on the time axis. Figs. 9A to 9C are views showing the values of substance information corresponding to three frames of substance information when the substance information correction unit 104 corrects the substance information of an abnormal pixel by using substance information of pixels adjacent to the abnormal pixel in the time direction. Fig. 9A shows the (m-1)th frame. Fig. 9B shows the mth frame. Fig. 9C shows the (m+1)th frame. Assume that a pixel 901 in the mth frame is an abnormal pixel.

In this modification, the substance information correction unit 104 performs correction by using preceding and succeeding frames in the time direction of the frame in which an abnormal pixel appears. A value after the correction can be calculated according to equation (5) given below.

$$Z_{eff\_m} = \frac{Z_{eff_{m-1}} + Z_{eff_{m+1}}}{2} \qquad \qquad ...(5)$$

where $Z_{eff\_m}$ is the effective atomic number of the mth frame after correction, $Z_{eff\_m-1}$ is the effective atomic number of the (m-1)th frame, and $Z_{eff\_m+1}$ is the effective atomic number of the (m+1)th frame. In the case shown in Figs. 9A to 9C, the substance information correction unit 104 can calculate the corrected value of the abnormal pixel 901 by using pixels 902 and 903. Performing correction by using preceding and succeeding frames in this manner can perform correction without damaging sharpness when the movement of the object is small.

Although this modification performs correction by using preceding and succeeding frames, the substance information correction unit 104 can also perform correction by using only the preceding frame or succeeding frame. For example, the substance information correction unit 104 can use a plurality of frames instead of only one frame. For example, when performing correction by using three frames before the mth frame, the substance information correction unit 104 can calculate the effective atomic number of the mth frame by adding the values obtained by respectively multiplying the effective atomic numbers of the (m-3)th, (m-2)th, and (m-1)th frames by coefficients of 0.1, 0.4, and 0.5.

Using preceding frames in this manner makes it possible to perform correction without waiting for the acquisition of succeeding frames. Although this modification has exemplified the correction using only fames on the time axis, it is also possible to calculate a corrected pixel by combining values on the time axis and in space. This system can also be configured to allow the operator to separately set a correction method via the operation unit 204 or to automatically set it.

[Modification 1-3]

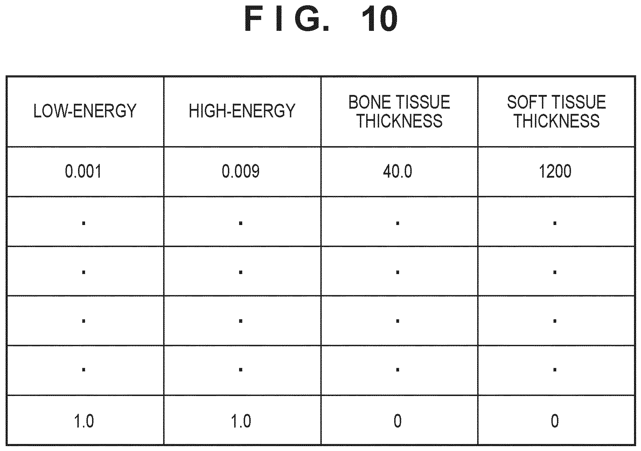

Although the first embodiment uses an effective atomic number as substance information, the thickness information of two types of substances designated in advance may be used as substance information. When, for example, bone and soft tissue are designated as two types of substances, it is possible to calculate images of the bone and the soft tissue. Fig. 10 shows an example of a conversion table according to this modification. Referring to Fig. 10, the first and second columns of the conversion table respectively describe low-energy pixel values and high-energy pixel values, and the third and fourth columns respectively describe the thicknesses of the bone tissue and the soft tissue. Values described in the conversion table can be calculated in advance based on equation (6) given below.

$$I_{correct} = \int E\, e^{-(\mu_{bone}(E)t_{bone} + \mu_{tissue}(E)t_{tissue})} dE \qquad ...(6)$$

where $I_{correct}$ is an image corresponding to an image after correction based on equation (1), E is the energy of radiation, $\mu_{bone}(E)$ is a linear attenuation coefficient set when the bone is irradiated with radiation having the energy E, $t_{bone}$ is the thickness of the bone, $\mu_{tissue}(E)$ is a linear attenuation coefficient set when the soft tissue is irradiated with radiation having the energy E, and $t_{tissue}$ is the thickness of the soft tissue. Preparing such a table makes it possible to generate a table for each substance thickness.

A conversion table may be set in accordance with the possible thicknesses of the bone. For example, when the chest region is to be imaged, the thickness of the bone may be set to 0 mm to 40 mm, and the thickness of the soft tissue may be set to 0 mm to 1200 mm. Alternatively, the above setting values may be designated in accordance with the physique of a patient. Physique measurement may be performed by, for example, a method using a measure or a method of estimating from a pixel value profile and the like.

As described above, according to this modification, the abnormal pixel detection unit 505 detects whether there is any abnormal pixel, based on whether the pixel values of a high-energy radiation image and a low-energy radiation image each do not fall within the range of the pixel values obtained from the thicknesses of substances that can be contained in the object at the time of imaging and two types of energies. In other words, according to this modification, the abnormal pixel detection unit 505 determines that there is an abnormal pixel, if the combination of two types of images after correction by the image correction unit 502 falls outside the conversion table shown in Fig. 10. This makes it possible to detect an abnormal pixel at the time of calculating substance information.

[Modification 1-4]

The first embodiment has exemplified the method of acquiring two types of energies by using the two sample/hold units. However, increasing the number of sample/hold operations can obtain information on more energies. For example, performing three sample/hold operations allows the image acquisition unit 501 to acquire radiation images obtained with three types of energies (a high-energy radiation image, intermediate-energy radiation image, and low-energy radiation image).

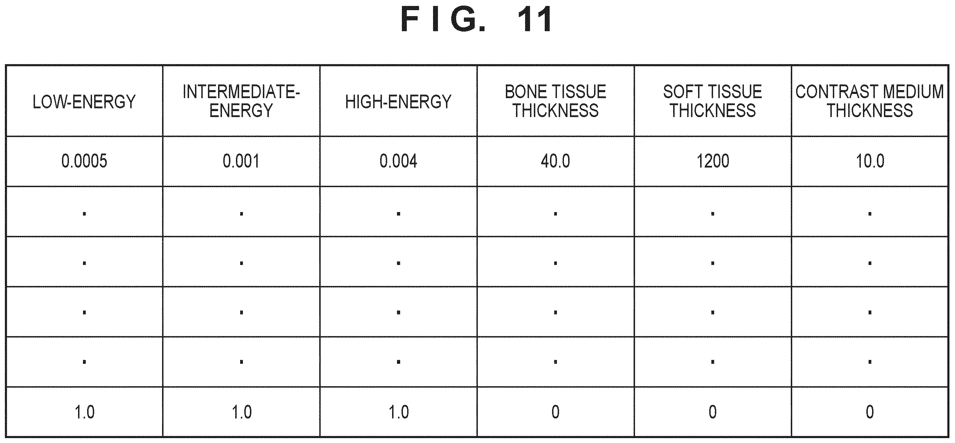

Fig. 11 shows an example of a conversion table according to this modification. Referring to Fig. 11, the first to third columns of the conversion table respectively describe pixel values of low-energy radiation images, the pixel values of intermediate-energy radiation images, and the pixel values of high-energy radiation images, and the fourth to sixth columns respectively describe the thicknesses of the bone tissue, the soft tissue, and the contrast medium. Values described in the conversion table can be calculated in advance based on equation (7) given below.

$$I_{correct} = \int E\, e^{-(\mu_{bone}(E)t_{bone} + \mu_{tissue}(E)t_{tissue} + \mu_{iodine}(E)t_{iodine})}\, dE$$

...(7)

where $I_{correct}$ is an image corresponding to an image after correction based on equation (1), E is the energy of radiation, $\mu_{bone}(E)$ is a linear attenuation coefficient set when the bone is irradiated with radiation having the energy E, tbone is the thickness of the bone, $\mu_{tissue}(E)$ is a linear attenuation coefficient set when the soft tissue is irradiated with radiation having the energy E, $t_{tissue}$ is the thickness of the soft tissue, $\mu_{iodine}(E)$ is a linear attenuation coefficient set when the contrast medium is irradiated with radiation having the energy E, and $t_{iodine}$ is the thickness of the contrast medium. In this case, a table concerning the contrast medium may be generated in accordance with the size of a contrast medium target. For example, when thin blood vessels like those in the heart or the like are contrast medium targets, the thicknesses may be set to 0 mm to 10 mm or the like.

According to this modification, the abnormal pixel detection unit 505 detects that there is an abnormal pixel, if the pixel values of a high-energy radiation image, an intermediate-energy radiation image, and a low-energy radiation image each do not fall within the range of the pixel values obtained from the thicknesses of substances that can be contained in the object at the time of imaging and three types of energies. In other words, according to this modification, the abnormal pixel detection unit 505 can detect an abnormal pixel at the time of calculating substance information by determining that there is an abnormal pixel if the combination of three types of images after correction by the image correction unit 502 falls outside the conversion table shown in Fig. 11.

As described above, according to the first embodiment and its modifications, it is possible to detect and correct an abnormal pixel in a plurality of radiation images obtained by radiation imaging with a plurality of different energies. Note that the various types of abnormal pixel detection methods described above can also be combined and implemented in the image processing apparatus 100. In addition, the substance information conversion unit 504 and the abnormal pixel detection unit 505 may concurrently perform processing or the abnormal pixel detection unit 505 may perform processing first.

[Second Embodiment]

(Arrangement of Radiation Imaging System 1200)

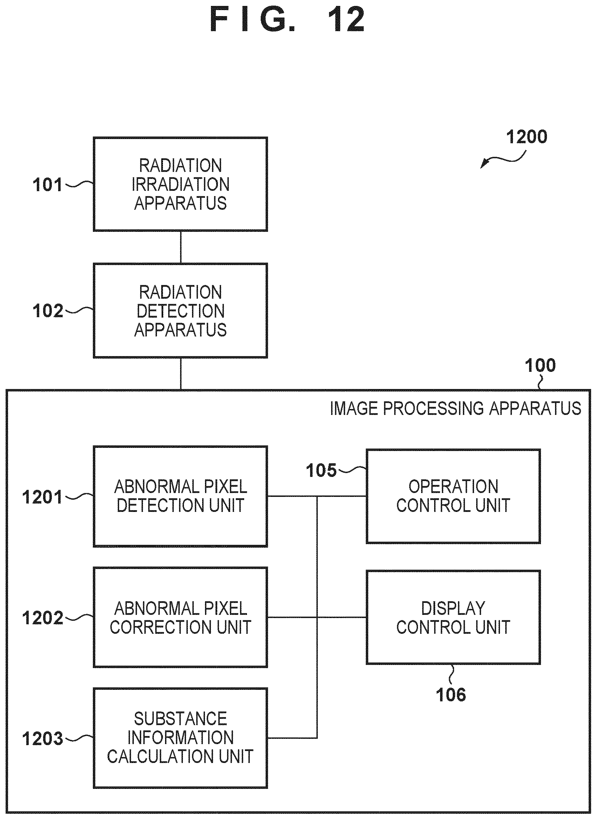

Fig. 12 shows an example of the arrangement of a radiation imaging system 1200 according to the second embodiment. A description of arrangements similar to those in Fig. 1 will be omitted. Assume that in this embodiment, as in the first embodiment, substance information includes thickness information for each effective atomic number or predetermined substance. An abnormal pixel detection unit 1201 detects the presence/absence of an abnormal pixel in each of two types of radiation images obtained by radiation imaging with radiation having two types of different energies. An abnormal pixel correction unit 1202 corrects an abnormal pixel detected by the abnormal pixel detection unit 1201. A substance information calculation unit 1203 calculates substance information based on the images obtained with a plurality of energies. The operations of the abnormal pixel detection unit 1201, the abnormal pixel correction unit 1202, and the substance information calculation unit 1203 will be described later.

(Abnormal Pixel Detection Method)

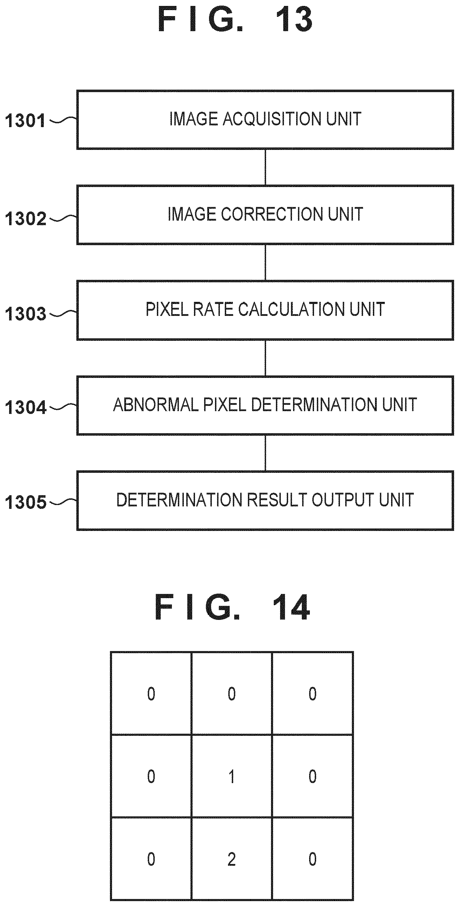

An abnormal pixel detection method in the abnormal pixel detection unit 1201 will be described next. Fig. 13 shows the functional arrangement of the abnormal pixel detection unit 1201. The abnormal pixel detection unit 1201 is constituted by an image acquisition unit 1301, an image correction unit 1302, a pixel rate calculation unit 1303, an abnormal pixel determination unit 1304, and a determination result output unit 1305. The image acquisition unit 1301 acquires a high-energy radiation image and a low-energy radiation image from the radiation detection apparatus 102. The image correction unit 1302 corrects the high-energy radiation image and the low-energy radiation image. A correction method is similar to that for processing by the image correction unit 502 according to the first embodiment, and hence a description of the method will be omitted. Subsequently, the pixel rate calculation unit 1303 calculates the pixel rate between the high-energy radiation image and the low-energy radiation image. A calculation method is similar to that in the pixel rate calculation unit 503 according to the first embodiment, and hence a description of the method will be omitted.

The abnormal pixel determination unit 1304 then determines, based on the pixel rate, which one of the high-energy radiation image and the low-energy radiation image includes an abnormal pixel. More specifically, the abnormal pixel determination unit 1304 compares the minimum value and the maximum value of the rate between a linear attenuation coefficient set when a substance that can be contained in an object is irradiated with radiation having higher energy at the time of imaging and a linear attenuation coefficient set when the substance is irradiated with radiation having lower energy. Unlike the first embodiment, the second embodiment need not use any conversion table (Fig. 6 or the like) for converting a pixel rate into substance information, and uses only the minimum pixel rate and the maximum pixel rate in the conversion table.

For example, the conversion table shown in Fig. 6 shows the maximum pixel rate and the minimum pixel rate that can occur under imaging conditions at the time of acquiring images. Accordingly, the abnormal pixel determination unit 1304 can determine the presence of an abnormal pixel if the pixel rate is larger than the maximum pixel rate or smaller than the minimum pixel rate. If the pixel rate is larger than the maximum pixel rate, it indicates that the pixel value of the low-energy image is large, and hence the abnormal pixel determination unit 1304 can determine that there is an abnormal pixel in the low-energy radiation image. In contrast to this, if the pixel rate is smaller than the minimum pixel rate, it indicates that the pixel value of the high-energy image is large, and hence the abnormal pixel determination unit 1304 can determine that there is an abnormal pixel in the high-energy radiation image. Discriminating whether a given pixel rate falls within or without the range makes it possible to output a result indicating which one of the high-energy radiation image and the low-energy radiation image includes an abnormal pixel.

The determination result output unit 1305 outputs the determination result obtained by the abnormal pixel determination unit 1304. Fig. 14 shows an example of the determination result generated by the determination result output unit 1305. A pixel having a pixel value of 0 is a normal pixel. A pixel having a pixel value of 1 is an abnormal pixel in a high-energy radiation image. A pixel having a pixel value of 2 is an abnormal pixel in a low-energy radiation image. Accordingly, for example, the determination result output unit 1305 sets and outputs a different numerical value for each pixel in accordance with a detection result concerning an abnormal pixel. This processing makes it possible to detect an abnormal pixel and discriminate which one of a high-energy radiation image and a low-energy radiation image includes an abnormal pixel.

(Method of Correcting Abnormal Pixel)

A method of correcting an abnormal pixel in the abnormal pixel correction unit 1202 will be described next. Fig. 16 shows the functional arrangement of the abnormal pixel correction unit 1202. The abnormal pixel correction unit 1202 is constituted by an image acquisition unit 1601, a determination result acquisition unit 1602, a pixel value correction unit 1603, and a corrected image output unit 1604. The abnormal pixel correction unit 1202 corrects the pixel values of a high-energy radiation image and a low-energy radiation image based on the determination result obtained by the abnormal pixel determination unit 1304.

The image acquisition unit 1601 acquires a high-energy radiation image and a low-energy radiation image. The determination result acquisition unit 1602 acquires a determination result from the determination result output unit 1305. The pixel value correction unit 1603 corrects a high-energy radiation image and a low-energy radiation image based on the acquired determination result. These images can be corrected by using a method using neighboring pixels in a space for each image or a method using pixels adjacent to each other in the time direction as in modification 1-2. Correcting each of a high-energy radiation image and a low-energy radiation image in this manner can correct only an abnormal pixel. Finally, the corrected image output unit 1604 outputs each of the corrected high-energy radiation image and the corrected low-energy radiation image. Performing such processing can acquire substance information having undergone correction of an abnormal pixel.

The substance information calculation unit 1203 acquires the corrected high-energy radiation image and the corrected low-energy radiation image from the corrected image output unit 1604, and calculates substance information. Substance information is calculated by a method using the conversion table shown Fig. 6 or the like as in the first embodiment.

As described above, the radiation imaging system 1200 according to this embodiment can obtain substance information with reduced noise by detecting and correcting an abnormal pixel from two image data with different energies and then calculating substance information.

[Modification 2-1]

The second embodiment has exemplified the method of acquiring radiation images obtained by radiation imaging with two types of energies by using the two sample/hold units. However, increasing the number of sample/hold operations can obtain more radiation images. For example, performing three sample/hold operations allows the image acquisition unit 1301 to acquire three radiation images, namely a high-energy radiation image, intermediate-energy radiation image, and low-energy radiation image.

This modification is configured to detect whether three acquired radiation images each include an abnormal pixel and correct the abnormal pixel. The pixel rate calculation unit 1303 calculates the rates of at least two of the pixel values of a high-energy radiation image, an intermediate-energy radiation image, and a low-energy radiation image for each pixel as two pixel rates. The abnormal pixel determination unit 1304 then determines the presence/absence of an abnormal pixel in the high-energy radiation image, the intermediate-energy radiation image, or the low-energy radiation image by comparing the two pixel rates with the minimum value and the maximum value of the rate between linear attenuation coefficients set in the case of a substance that can be contained in the object and higher energy at the time of imaging and in the case of the substance and lower energy.

For example, the pixel rate calculation unit 1303 calculates the pixel rate between the high-energy radiation image and the low-energy radiation image and the pixel rate between the high-energy radiation image and the intermediate-energy radiation image as in the second embodiment. The abnormal pixel determination unit 1304 then determines whether each of the calculated pixel rates falls within the range from the minimum pixel rate to the maximum pixel rate. Assume that the abnormal pixel determination unit 1304 calculates both the pixel rates with the high-energy radiation image as a denominator. In this case, if the pixel rate is smaller than the minimum pixel rate, the abnormal pixel determination unit 1304 determines that there is an abnormal pixel in the high-energy radiation image. If the pixel rate is larger than the maximum pixel rate, the abnormal pixel determination unit 1304 determines that there is an abnormal pixel in the intermediate-energy radiation image or the low-energy radiation image. Detecting an abnormal pixel by calculating each rate in this manner can cope with two or more energy images.

As described above, according to the second embodiment and its modification, it is possible to efficiently detect whether there is any abnormal pixel in any of a plurality of radiation images obtained by radiation imaging with a plurality of different energies and to correct an abnormal pixel, if any.

[Other Embodiments]

The present invention can also be implemented by supplying a program for implementing one or more functions of each embodiment described above to a system or apparatus via a network or a storage medium and causing one or more processors in the system or apparatus to read and execute the program. The present invention can also be implemented by a circuit implementing one or more functions (for example, ASIC).

The present invention is not limited to the above-described embodiments, and various changes and modifications can be made within the spirit and scope of the present invention. Therefore, to apprise the public of the scope of the present invention, the following claims are made.

**Claims**

1. An image processing apparatus **characterized by** comprising:

   image acquisition means for acquiring a plurality of radiation images by radiation imaging with a plurality of energies; and
   detection means for detecting, based on pixel values of the plurality of radiation images, whether there is any abnormal pixel in at least one of the plurality of radiation images.

2. The image processing apparatus according to claim 1, **characterized in that** the image acquisition means acquires a high-energy radiation image as a radiation image obtained by radiation imaging with high energy and a low-energy radiation image as a radiation image obtained by radiation imaging with low energy, which are radiation images obtained by radiation imaging with two types of energies, and
   the detection means detects whether there is the abnormal pixel in any of the high-energy radiation image and the low-energy radiation image, based on a pixel value of the high-energy radiation image, a pixel value of the low-energy radiation image, and information concerning a predetermined substance that can be contained in an object at the time of radiation imaging.

3. The image processing apparatus according to claim 2, **characterized in that** the detection means detects that the abnormal pixel is present, if a pixel value of the high-energy radiation image and a pixel value of the low-energy radiation image each do not fall within a range of pixel values obtained from effective atomic numbers of substances that can be contained in the object at the time of imaging and the two types of energies.

4. The image processing apparatus according to claim 2, **characterized in that** the detection means detects that the abnormal pixel is present, if a pixel value of the high-energy radiation image and a pixel value of the low-energy radiation image each do not fall within a range of pixel values obtained from thicknesses of substances that can be contained in the object at the time of imaging and the two types of energies.

5. The image processing apparatus according to claim 2, **characterized in that** the image acquisition means acquires the high-energy radiation image, the low-energy radiation image, and an intermediate-energy radiation image as a radiation image obtained by radiation imaging with an energy between the high energy and the low energy, which are radiation images obtained by radiation imaging with three types of energies, and
the detection means detects that the abnormal pixel is present, if a pixel value of the high-energy radiation image, a pixel value of the intermediate-energy radiation image, and a pixel value of the low-energy radiation image each do not fall within a range of pixel values obtained from thicknesses of substances that can be contained in the object at the time of imaging and the three types of energies.

6. The image processing apparatus according to any one of claims 2 to 5, **characterized by** further comprising calculation means for calculating a rate between a pixel rate of the high-energy radiation image and a pixel value of the low-energy radiation image for each pixel.

7. The image processing apparatus according to claim 6, **characterized in that** the detection means detects that the abnormal pixel is present if the pixel rate does not fall within a range of a rate between a linear attenuation coefficient set when a substance that can be contained in the object with radiation having the high energy at the time of imaging and a linear attenuation coefficient set when the substance is irradiated with radiation having the low energy.

8. The image processing apparatus according to claim 6 or 7, **characterized by** further comprising:

   substance information conversion means for converting the pixel rate into substance information specifying a substance that can be contained in the object at the time of imaging; and
   substance information correction means for correcting the substance information of the abnormal pixel detected by the detection means.

9. The image processing apparatus according to claim 8, **characterized in that** the substance information correction means corrects the substance information of the abnormal pixel by using the substance information of pixels adjacent to the abnormal pixel in spatial directions.

10. The image processing apparatus according to claim 8 or 9, **characterized in that** the substance information correction means corrects the substance information of the abnormal pixel by using the substance information of pixels adjacent to the abnormal pixel in a time direction.

11. The image processing apparatus according to any one of claims 8 to 10, **characterized in that** the substance information is an effective atomic number corresponding to the substance or a thickness of the substance.

12. The image processing apparatus according to claim 1, **characterized in that** the image acquisition means acquires a high-energy radiation image as a radiation image obtained by radiation imaging with high energy and a low-energy radiation image as a radiation image obtained by radiation imaging with low energy, which are radiation images obtained by radiation imaging with two types of energies, and
the apparatus further comprises
calculation means for calculating a rate between a pixel value of the high-energy radiation image and a pixel value of the low-energy radiation image, and
determination means for determining whether there is any abnormal pixel in the high-energy radiation image or the low-energy radiation image by comparing the rate with a minimum value and a maximum value of a rate between a linear attenuation coefficient set when a substance that can be contained in an object with radiation having the high energy at the time of imaging and a linear attenuation coefficient set when the substance is irradiated with radiation having the low energy.

13. The image processing apparatus according to claim 12, **characterized by** further comprising abnormal pixel correction means for correcting each of pixel values of the high-energy radiation image and the low-energy radiation image based on a determination result obtained by the determination means.

14. The image processing apparatus according to claim 12, **characterized in that** the image acquisition means further acquires an intermediate-energy radiation image that is a radiation image obtained by radiation imaging with energy between the high energy and the low energy,
the calculation means calculates, for each pixel, a pixel rate of at least two of a pixel value of the high-energy radiation image, a pixel value of the intermediate-energy radiation image, and a pixel value of the low-energy radiation image, and
the determination means determines whether there is any abnormal pixel in the high-energy radiation image, the intermediate-energy radiation image, or the low-energy radiation image by comparing the pixel rate between the two pixel values with a minimum value and a maximum value of a rate between a linear attenuation coefficient set when a substance that can be contained in the object with radiation having the high energy at the time of imaging and a linear attenuation coefficient set when the substance is irradiated with radiation having the low energy.

15. The image processing apparatus according to claim 14, **characterized by** further comprising abnormal pixel correction means for correcting each of pixel values of the high-energy radiation image, the intermediate-energy radiation image, and the low-energy radiation image based on a determination result obtained by the determination means.

16. The image processing apparatus according to claim 13 or 15, **characterized in that** the abnormal pixel correction means corrects a pixel value of the abnormal pixel by using pixel values of pixels adjacent to the abnormal pixel in spatial directions.

17. The image processing apparatus according to claim 13 or 15, **characterized in that** the abnormal pixel correction means corrects a pixel value of the abnormal pixel by using pixel values of pixels adjacent to the abnormal pixel in a time direction.

18. The image processing apparatus according to any one of claims 13 and 15 to 17, **characterized by** further comprising substance information conversion means for converting to substance information concerning a substance that can be contained in the object at the time of imaging,
wherein the calculation means further calculates a rate of a pixel value of an image corrected by the abnormal pixel correction means as a corrected pixel rate, and
the substance information conversion means converts the corrected pixel rate into substance information concerning a substance that can be contained in the object at the time of imaging.

19. The image processing apparatus according to claim 18, **characterized in that** the substance information is an effective atomic number corresponding to the substance or a thickness of the substance.

20. An image processing method **characterized by** comprising:

an image acquisition step of acquiring a plurality of radiation images by radiation imaging with a plurality of energies; and
a detection step of detecting, based on pixel values of the plurality of radiation images, whether there is any abnormal pixel in at least one of the plurality of radiation images.

21. A radiation imaging apparatus **characterized by** comprising:

image acquisition means, including a phosphor configured to convert radiation into light and a detector configured to detect light, for acquiring a plurality of radiation images by radiation imaging with a plurality of energies; and
detection means for detecting, based on pixel values of the plurality of radiation images, whether radiation that is not converted into light by the phosphor has entered the detector.

22. A method of controlling a radiation imaging apparatus including a phosphor configured to convert radiation into light and a detector configured to detect light, the method comprising:

an image acquisition step of acquiring a plurality of radiation images by radiation imaging with a plurality of energies; and
a detection step of detecting, based on pixel values of the plurality of radiation images, whether light that is not converted into light by the phosphor has entered the detector.

23. A program for causing a computer to function as an image processing apparatus defined in any one of claims 1 to 19.

24. A program for causing a computer to function as a radiation imaging apparatus defined in claim 21.

# FIG. 1

101 — RADIATION IRRADIATION APPARATUS

— 10

102 — RADIATION DETECTION APPARATUS

100

IMAGE PROCESSING APPARATUS

103 — SUBSTANCE INFORMATION CALCULATION UNIT

105 — OPERATION CONTROL UNIT

104 — SUBSTANCE INFORMATION CORRECTION UNIT

106 — DISPLAY CONTROL UNIT

# F I G. 2

100

| | | | |
|---|---|---|---|
| 201 | CONTROL UNIT | OPERATION UNIT | 204 |
| 202 | STORAGE UNIT | DISPLAY UNIT | 205 |
| 203 | COMMUNICATION UNIT | | |

FIG. 3

RADIATION SIGNAL ACQUISITION UNIT — 301

3011

PHOSPHOR

3012

PHOTODETECTOR

FIRST SAMPLE/HOLD UNIT — 303

RESET UNIT — 302

READOUT UNIT — 305

SECOND SAMPLE/HOLD UNIT — 304

DIFFERENCE PROCESSING UNIT — 306

EP 3 708 082 A1

**F I G.  4**

EP 3 708 082 A1

# F I G. 5

| | |
|---|---|
| 501 | IMAGE ACQUISITION UNIT |
| 502 | IMAGE CORRECTION UNIT |
| 503 | PIXEL RATE CALCULATION UNIT |
| 504 | SUBSTANCE INFORMATION CONVERSION UNIT |
| 505 | ABNORMAL PIXEL DETECTION UNIT |
| 506 | PIXEL ADJUSTMENT UNIT |
| 507 | SUBSTANCE INFORMATION OUTPUT UNIT |

# F I G. 6

# F I G. 7

# F I G. 8A

| 8.1 | 12 | 12 |
|-----|-----|-----|
| 7.8 | 0 | 7.8 |
| 8.0 | 7.9 | 7.8 |

# F I G. 8B

| 8.1 | 12 | 12 |
|-----|-----|-----|
| 7.8 | 0 | 7.8 |
| 8.0 | 0 | 7.8 |

F I G.  9A

902

| 8.1 | 12 | 12 |
| 7.8 | 7.8 | 7.8 |
| 8.0 | 7.9 | 7.8 |

F I G.  9B

901

| 8.1 | 12 | 12 |
| 7.8 | 0 | 7.8 |
| 8.0 | 7.9 | 7.8 |

F I G.  9C

903

| 8.1 | 12 | 12 |
| 7.8 | 7.8 | 7.8 |
| 8.0 | 7.9 | 7.8 |

# FIG. 10

| LOW-ENERGY | HIGH-ENERGY | BONE TISSUE THICKNESS | SOFT TISSUE THICKNESS |
|---|---|---|---|
| 0.001 | 0.009 | 40.0 | 1200 |
| . | . | . | . |
| . | . | . | . |
| . | . | . | . |
| . | . | . | . |
| 1.0 | 1.0 | 0 | 0 |

# FIG. 11

| LOW-ENERGY | INTERMEDIATE-ENERGY | HIGH-ENERGY | BONE TISSUE THICKNESS | SOFT TISSUE THICKNESS | CONTRAST MEDIUM THICKNESS |
|---|---|---|---|---|---|
| 0.0005 | 0.001 | 0.004 | 40.0 | 1200 | 10.0 |
| . | . | . | . | . | . |
| . | . | . | . | . | . |
| . | . | . | . | . | . |
| . | . | . | . | . | . |
| 1.0 | 1.0 | 1.0 | 0 | 0 | 0 |

EP 3 708 082 A1

# F I G. 12

**1200**

**101**— RADIATION IRRADIATION APPARATUS

**102**— RADIATION DETECTION APPARATUS

**100**

IMAGE PROCESSING APPARATUS

**1201**— ABNORMAL PIXEL DETECTION UNIT

**105**— OPERATION CONTROL UNIT

**1202**— ABNORMAL PIXEL CORRECTION UNIT

DISPLAY CONTROL UNIT

**106**

**1203**— SUBSTANCE INFORMATION CALCULATION UNIT

# F I G. 13

| | |
|---|---|
| 1301 | IMAGE ACQUISITION UNIT |
| 1302 | IMAGE CORRECTION UNIT |
| 1303 | PIXEL RATE CALCULATION UNIT |
| 1304 | ABNORMAL PIXEL DETERMINATION UNIT |
| 1305 | DETERMINATION RESULT OUTPUT UNIT |

# F I G. 14

| 0 | 0 | 0 |
|---|---|---|
| 0 | 1 | 0 |
| 0 | 2 | 0 |

# F I G. 15

| LOW-ENERGY | HIGH-ENERGY | EFFECTIVE ATOMIC NUMBER |
|---|---|---|
| 0.1 | 0.09 | 20.0 |
| . | . | . |
| . | . | . |
| . | . | . |
| . | . | . |
| 0.8 | 0.9 | 5.0 |

# F I G. 16

**1601**

IMAGE ACQUISITION UNIT

**1602**

DETERMINATION RESULT ACQUISITION UNIT

**1603** PIXEL VALUE CORRECTION UNIT

**1604** CORRECTED IMAGE OUTPUT UNIT

## INTERNATIONAL SEARCH REPORT

| | International application No. |
|---|---|
| | PCT/JP2018/033731 |

**A. CLASSIFICATION OF SUBJECT MATTER**
Int. Cl. A61B6/00(2006.01)i, A61B6/03(2006.01)i

According to International Patent Classification (IPC) or to both national classification and IPC

**B. FIELDS SEARCHED**

Minimum documentation searched (classification system followed by classification symbols)
Int. Cl. A61B6/00, A61B6/03

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2012-134781 A (KONICA MINOLTA MEDICAL & | 1, 20-24 |
| A | GRAPHIC, INC.) 12 July 2012, paragraphs [0066]- | 2-19 |
| | [0068], [0084]-[0094] (Family: none) | |
| A | JP 11-205682 A (FUJI PHOTO FILM CO., LTD.) 30 July 1999, paragraphs [0021]-[0055] & US 6125166 A, column 10, line 16 to column 16, line 19 | 1-24 |

☐ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07.12.2018 | 18.12.2018 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

27

**EP 3 708 082 A1**

**Patent documents cited in the description**

- JP 2014183475 A